# EUROPEAN PATENT SPECIFICATION

(11) **EP 4 260 817 B1**
(45) Date of publication and mention of the grant of the patent: **08.07.2026**
(21) Application number: 21902338.9
(22) Date of filing: 16.11.2021
(51) Int. Cl.: A61B 17/12, A61B 90/00

(54) **OCCLUDING DEVICE**
VERSCHLUSSVORRICHTUNG
DISPOSITIF D'OCCLUSION

(30) Priority: 10.12.2020 CN 202011436103
(43) Date of publication of application: 18.10.2023
(73) Proprietor: Lifetech Scientific (Shenzhen) Co., Ltd., Shenzhen, Guangdong 518063 (CN)
(72) Inventor: LI, Anning, Shenzhen, Guangdong 518063 (CN); LIU, Jianyong, Shenzhen, Guangdong 518063 (CN)
(74) Representative: Michalski Hüttermann & Partner mbB
(86) International application number: PCT/CN2021/130920
(87) International publication number: WO 2022/121631

(56) References cited:
- CN-A- 102 805 654
- CN-A- 106 923 883
- CN-A- 107 847 232
- CN-A- 108 926 368
- CN-A- 110 522 486
- CN-U- 206 792 446
- CN-U- 211 325 298
- DE-A1- 102016 012 395
- US-A1- 2011 054 519
- US-A1- 2019 083 075

## Description

### TECHNICAL FIELD

The embodiments relate to the field of interventional medical instruments and an occlusion device.

### BACKGROUND

As for the existing umbrella-shaped left atrial appendage occluder, each of supporting bodies on a fixing disc is a single rod independent from each other and suspended, and the fixing disc is formed by cutting metal alloy tubes to form each of supporting bodies before heat-setting. Therefore, the supporting force of the umbrella-shaped left atrial appendage occluder to the inner wall of the left atrial appendage is relatively large. In addition, since the ravines between pectinate muscles in the left atrial appendage are weakest and irregularly distributed, it is possible that the single rod on the fixing disc will abut against these ravines upon release and spreading. As the heart continues to contract and relax, these single rods tend to grind or rupture the left atrial appendage at the ravines, causing symptoms such as pericardial effusion and endangering the patient's life.

D1 (US 2019/0083075 A1) discloses an example occlusive implant. The example occlusive implant includes an expandable framework configured to shift between a collapsed configuration and an expanded configuration, an occlusive member disposed along at least a portion of the expandable framework and a sealing member disposed along the occlusive member.

CN 108926368 A discloses a left aurcle plugging device adopting a staggered connection structure and an assembly method of the left aurcle plugging device. The left aurcle plugging device comprises a sealing part and an anchoring part which are connected with each other, each of the sealing part and the anchoring part is provided with a connecting part formed through shape convergence, and the connecting parts of the sealing part and the anchoring part extend opposite to each other and realize staggered fixing; and the anchoring part is formed through cutting, and the far-end side of the anchoring part is of a closed ring structure before release. For the left aurcle plugging device provided by the invention, the connecting method of the sealing part and the anchoring part is improved, so that the integral axial elongation of the left aurcle plugging device can be avoided and delayed, and further, the sealing part attaches to and plugs the left aurcle opening.

### SUMMARY

Based on this, an improved occlusion device is desired to address the problem that the independent and suspended single rod of the existing left atrial appendage occluder is easy to wear or break through the left atrial appendage in the groove between the pectinate muscles. The invention is defined in the appended set of claims.

An occlusion device includes a fixing portion. The fixing portion includes a plurality of supporting bodies. The plurality of supporting bodies includes a plurality of supporting sections spaced apart along a circumferential direction of the fixing portion. For each of the plurality of supporting sections, tail ends of at least two adjacent supporting sections bend and extend towards the interior of the fixing portion to form a convergence section, by which the tail ends of the at least two adjacent supporting sections are connected. The at least two adjacent supporting sections and the corresponding convergence section form a set of suspended sections, all sets of suspended sections are spaced apart, suspended along the circumferential direction of the fixing portion, and being independent from each other.

In one embodiment, the plurality of supporting sections inclines circumferentially around a central axis of the fixing portion.

In one embodiment, at least two of the supporting sections within at least one set of the suspended sections incline in the same or different directions along the circumferential direction of the fixing portion.

In one embodiment, at least two adjacent sets of the suspended sections incline in different directions along the circumferential direction of the fixing portion.

In one embodiment, each of the supporting sections includes a first supporting area and a second supporting area connected to each other; the first supporting area deforms along the circumferential direction of the fixing portion upon release of the fixing portion; and a maximum width of the first supporting area along the circumferential direction of the fixing portion after deformation is greater than a maximum width of the second supporting area along the circumferential direction of the fixing portion.

In one embodiment, the second supporting area deforms along the circumferential direction of the fixing portion upon release of the fixing portion.

In one embodiment, an innermost end portion of the convergence section is connected with an internally hollow woven mesh.

In one embodiment, the occlusion device further includes a sealing portion connected to the fixing portion, where one end of each of the plurality of supporting bodies is connected to the sealing portion and the other end extends away from the sealing portion.

In one embodiment, the fixing portion further includes a central end portion, and the occlusion device further includes a sealing portion connected to the central end portion and disposed at one side of the fixing portion; one end of each of the plurality of supporting bodies is connected to the central end portion, and the other end extends radially outward from the central end portion and flipping towards the sealing portion so as to form an flipping section; each of the plurality of supporting bodies continues to extend from a tail end of the flipping section towards the sealing portion so as to form the supporting section.

In one embodiment, the sealing portion includes a distal disc surface facing the fixing portion, at least a part of the distal disc surface being coated with at least one thin-film body.

Compared with the related art, the suspended sections of the above occlusion device retain the advantages of being independent from each other and with a large active space circumferentially of single supporting bodies circumferentially spaced apart and suspended, which can adaptively deform according to different internal tissue structures, and thus has flexible environmental adaptability. In addition, the suspended sections enable the tail ends of two adjacent supporting sections to be connected to form a closed loop structure bending towards the interior of the fixing portion, which not only has a better anti-damage effect, but also can prevent a single supporting section from being locally deformed to a too large extent, and buffer the supporting section with a single rod and the ends thereof being not closed from greater pressure on the inner wall of the left atrial appendage, so as to enhance the fatigue resistance of the occlusion device, and at the same time, reduce the stimulation, wear, or even rupture to the inner wall of the left atrial appendage.

### BRIEF DESCRIPTION OF THE DRAWINGS

FIG. 1 is an overall structural diagram of an occlusion device in Embodiment 1;
FIG. 2 is a structural diagram of a fixing portion in FIG. 1;
FIG. 3 is another structural diagram of a convergence section in Embodiment 1;
FIG. 4 is another structural diagram of a convergence section in Embodiment 1;
FIG. 5 is another structural diagram of a fixing portion in Embodiment 1;
FIG. 6 is another structural diagram of a fixing portion in Embodiment 1;
FIG. 7 is a structural diagram of a fixing portion in Embodiment 2;
FIG. 8 is another structural diagram of a fixing portion in Embodiment 2;
FIG. 9 is a structural diagram of one of supporting sections of a fixing portion in Embodiment 3;
FIG. 10 is another structural diagram of one of supporting sections of a fixing portion in Embodiment 3; and
FIG. 11 is an overall structural diagram of an occlusion device in Embodiment 4.

### DETAILED DESCRIPTION

In order to make the objects, solutions, and advantages of the embodiments clear, further detail with reference to the drawings and embodiments is provided. It is understood that the particular embodiments described herein are illustrative only and are not intended as limiting.

It is noted that in the field of interventional medical instruments, generally, an end of the medical instrument implanted in a human body or animal body, is defined as a "proximal end" in response to being closer to an operator, and an end is defined as a "distal end" in response to being farther from the operator, where the "proximal end" and "distal end" of any component of medical instruments are defined according to this principle. The term "axial" generally refers to a length direction of the medical instruments when they are transported, and the term "radial" generally refers to the direction of medical instruments perpendicular to their "axial direction," where "axial" and "radial" of any component of the medical instruments are defined according to this principle. The term "connect" referred to in embodiments includes instances where two components are directly connected to each other and indirectly connected via other components. While not specifically stated, the following description is directed to the device in its natural state free from external forces or upon release and spreading in the body.

The solutions are further explained in detail in combination with embodiments as follows.

### Embodiment 1

The occlusion device according to Embodiment 1 may be used to occlude the left atrial appendage as well as other internal tissues with openings, such as atrial septal defects. The occlusion device is hereinafter described in detail using the occluding of the left atrial appendage as an example.

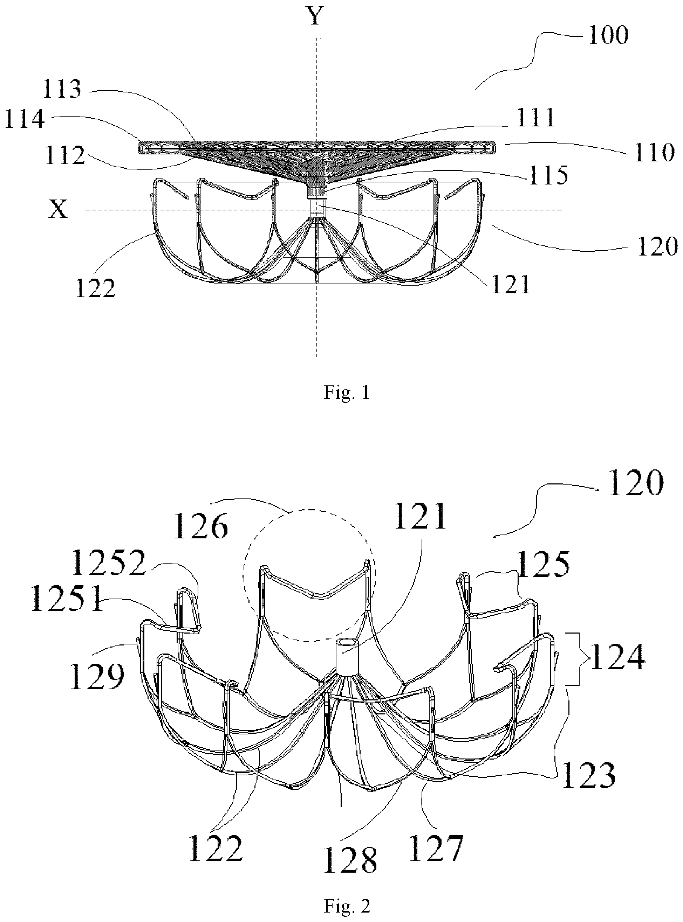

Referring to FIG. 1, an occlusion device 100 includes a fixing portion 120 and a sealing portion 110 connected to the fixing portion 120. The sealing portion 110 and the fixing portion 120 are spaced apart along an axial direction Y of the occlusion device 100. The sealing portion 110 is disposed at a proximal end of the occlusion device 100, and the fixing portion 120 is disposed at a distal end of the occlusion device 100. The occlusion device 100 has a compressed state contained in a sheath to facilitate transportation, and an expanded state after extending from the distal end of the sheath and expanding from expansion as shown in FIG. 1. The shape of the occlusion device 100 is completely or substantially the same as in FIG. 1 after release and deploying in the left heart ear. In other embodiment, for occluding atrial septal defects, the sealing portion 110 and the fixing portion 120 may abut against each other upon release to fix the occlusion device 100 onto the spacing between the left atrium and the right atrium.

The sealing portion 110 is woven into a mesh tube by a plurality of woven wires 111, and end portions of the woven wires 111 are closed and fixed by a sleeve at both ends of the mesh tube. The mesh tube is then heat-set into a disc, column, or plug shape to obtain the sealing portion 110 for occluding the opening of the left atrial appendage. The sealing portion 110 includes a distal disc surface 112 facing the fixing portion 120, and a proximal disc surface 113 opposite to the distal disc surface 112. At least one thin-film body 114 is provided inside the sealing portion 110, and the edge of the thin-film body 114 is fixed on the woven wires 111 at the edge of the sealing portion 110. The thin-film body 114 serves to block the flow of blood from one side of the sealing portion 110 to the other side, and further to block the flow of blood between the left atrial appendage and the left atrium.

The fixing portion 120 includes a plurality of supporting bodies 122, and a central end portion 121, to which a distal sleeve 115 of the sealing portion 110 is connected. The supporting bodies 122 on the fixing portion 120 may be rods obtained by cutting metal alloy tubes or polymer tubes or may be rods made by weaving or winding woven wires.

As shown in FIGS. 1 and 2, each of proximal ends of the plurality of supporting bodies 122 is connected to the central end portion 121, and each of distal ends extends radially outward from the central end portion 121 and flipping towards the sealing portion 110 to form a flipping section 123. Each of the plurality of supporting bodies 122 continues to extend from a distal end of the flipping section 123 towards the sealing portion 110 to form a supporting section 124. As shown in FIG. 2, twelve supporting bodies 122 correspond to twelve supporting sections 124, and the twelve supporting sections 124 are spaced apart along a circumferential direction of the fixing portion 120. For two adjacent supporting bodies 122, their distal ends continue to bend and extend from tail ends of the corresponding two adjacent supporting sections 124 towards the interior of the fixing portion 120 to form a convergence section 125, by which the tail ends of the two supporting sections 124 are connected, such that distal tail ends of the two supporting bodies 122 are connected to form a closed tail end. The two adjacent supporting sections 124 and the convergence section 125 connected thereto form a set of suspended sections 126. Therefore, the fixing portion 120 is provided with six suspended sections 126, and all the suspended sections 126 are spaced apart and suspended along the circumferential direction of the fixing portion 120.

Compared with the related art, the suspended sections 126 retain the advantages of being independent from each other and with a large active space circumferentially of single supporting bodies circumferentially spaced apart and suspended, which can adaptively deform according to different internal tissue structures, and thus has flexible environmental adaptability. In addition, the suspended sections 126 enable the tail ends of two adjacent supporting sections 124 to be connected to form a closed loop structure bending towards the interior of the fixing portion 120, which not only has a better anti-damage effect, but also can prevent a single supporting section 124 from being locally deformed to a too large extent, and buffer the supporting section with a single rod and the ends thereof being not closed from greater pressure on the inner wall of the left atrial appendage, so as to enhance the fatigue resistance of the occlusion device 100, and at the same time, reduce the stimulation, wear, or even rupture to the inner wall of the left atrial appendage.

In the embodiment, each the supporting body 122 of the fixing portion 120 includes a lead-out section 127 radially radiating from the central end portion 121 towards the distal end, two branch sections 128 with a certain angle connected to a distal end of the lead-out section 127, and a supporting section 124 connected to the two branch sections 128 in the middle of two adjacent supporting bodies 122. Each of the lead-out sections 127 surrounds and radially radiates from the central end portion 121 towards the distal end, cooperating with each other to form a recessed area. A proximal end of each of the two branch sections 128 is connected to the distal end of the corresponding lead-out section 127, and a distal end of each of the two branch sections 128 flipping towards the direction of the sealing portion 110. The lead-out section 127 of each of the supporting bodies 122 and the two branch sections 128 connected thereto form part of the flipping section 123. Each of the supporting section 124 is parallel or approximately parallel to a central axis Y of the fixing portion 120. The supporting sections 124 may be provided with an anchor 129, and a distal end of the anchor 129 faces towards the sealing portion 110. The anchor 129 penetrates to internal tissue upon release of the fixing portion 120, to assist in fixing the occlusion device 100 and preventing the occlusion device 100 from being displaced. The fixing portion 120, with a plurality of branch sections 128, prevents the local region of the flipping section 123 from deforming excessively to affect the fixed effect, and also reduces the stimulation by the over-deformed portion to the internal tissue.

The convergence section 125 is V-shaped or U-shaped, or may be other shapes, as desired. One end of the convergence section 125 is connected to a distal end of one supporting section 124, and the other end of the convergence section 125 is connected to a distal end of another supporting section 124, and the two supporting sections 124 are adjacent to each other and disposed at the same suspended section 126. The convergence section 125 bends overall from the two end portions towards the interior of the fixing portion 120, to prevent an innermost end portion of the convergence section 125 from scratching the internal tissue when the fixing portion 120 spreads in the body. At the same time, it is possible to avoid a large local deformation of the single supporting section 124, buffering the greater pressure of the supporting section 124 on the inner wall of the left atrial appendage.

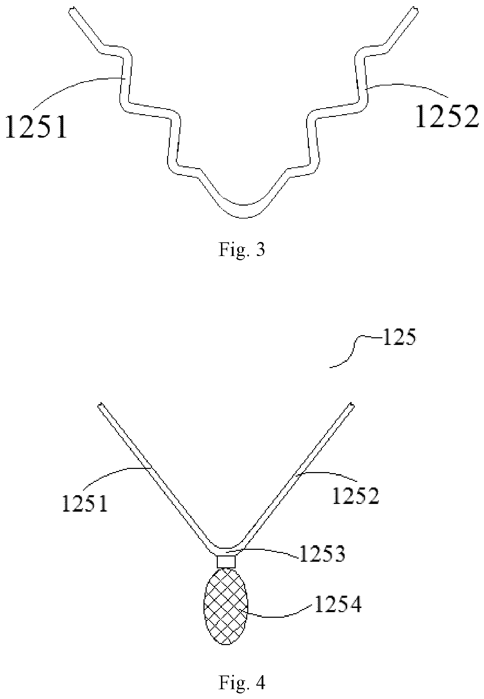

When the convergence section 125 is in a V-shape, it includes a first convergence rod 1251 and a second convergence rod 1252 connected to each other, where the first convergence rod 1251 and the second convergence rod 1252 are both linear rods. The convergence section 125 is in a U-shape, includes a first convergence rod 1251, a second convergence rod 1252, and a connecting rod connecting the first convergence rod 1251 and the second convergence rod 1252, where the first convergence rod 1251 and the second convergence rod 1252 are both linear rods, and the connecting rod is an arc-shaped rod. In another embodiment, as shown in FIG. 3, the first convergence rod 1251 and the second convergence rod 1252 are folded linear rods. In another embodiments, the first convergence rod 1251 and the second convergence rod 1252 may be wavy rods so they have a certain elastic deformation ability, which can improve the flexibility of the convergence section 125 and reduce the extrusion pressure and stimulation of the convergence section 125 against the tissue in the body.

Further, in other embodiments, at least a part of the distal disc surface 112 of the sealing portion 110 is coated with at least one thin-film body, the one thin-film body coating the entire region of the distal disc surface 112, or coating a part region opposite to one or more of convergence sections 125 in an axial direction; alternatively, the multilayer thin-film bodies may be superimposed or connected to each other to coat the entire region of the distal disc surface 112 or to coat the part region opposite to one or more of convergence sections 125 in an axial direction. In this way, the sealing property of the sealing portion 110 can be enhanced, and the convergence section 125 of the fixing portion 120 can be prevented from hooking to the sealing portion 110 and interfering with the proper performance of the sealing procedure.

In other embodiments, as shown in FIG. 4, an end of the convergence section 125 to which the first convergence rod 1251 and the second convergence rod 1252 are connected is an innermost end portion 1253 of the convergence section 125, and the innermost end portion 1253 is connected with a closed and internally hollow woven mesh 1254. The woven mesh 1254 may be spherical, cylindrical, or a non-closed mesh, which is used to reduce the extrusion pressure and stimulation, or injury, caused by the innermost end portion 1253 of the convergence section 125 when it contacts the internal tissue.

The occlusion device 100 may be configured to provide transportation and release using a sheath and a transporting rod. The proximal sleeve of the sealing portion 110 is detachably connected to a distal end of the transporting rod. During the occluding operation, the sealing portion 110 and the transporting rod are contained within the sheath after connected and are transported to the target position in the body along with the sheath. The transporting rod is then transported towards the distal end so that the fixing portion 120 extends from a distal end of the sheath for release. The transporting rod continues to be transported towards the distal end upon the fixing portion 120 releasing and being fixed at a target position in the body, so that the sealing portion 110 extends from the distal end of the sheath for release to occlude the opening in the internal tissue. Finally, the transporting rod is disconnected from the sealing portion 110, and the transporting rod and the sheath are retrieved outside the body, thereby completing the occluding operation.

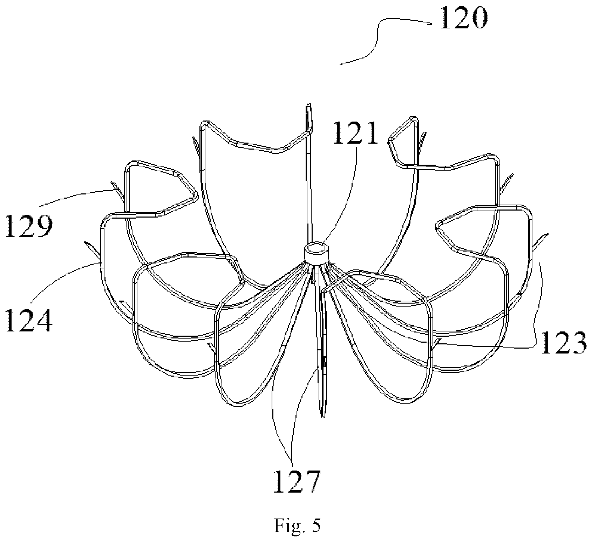

In other embodiments, the number of supporting bodies 122 may be set as desired, as may the number of branch sections 128 corresponding to each of the lead-out sections 127. For example, as shown in FIG. 5, each of the lead-out sections 127 surrounds and radially radiates from the central end portion 121 towards the distal end, cooperating with each other to form a recessed area, and then flipping towards the direction of the sealing portion 110 to form the flipping section 123. A proximal end of each of the supporting sections 124 is connected to the distal end of one of the corresponding lead-out sections 127 without the branch sections 128. Each of the supporting sections 124 may be provided with an anchor 129, and a distal end of the anchor 129 faces towards the sealing portion 110.

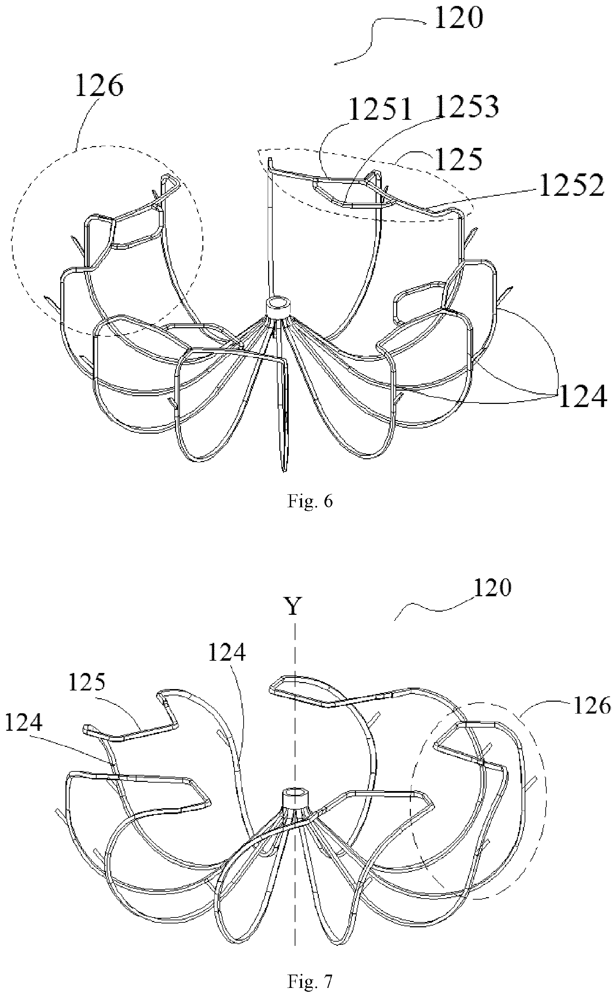

In other embodiments, as shown in FIG. 6, for three adjacent supporting bodies 122, their distal ends bend and extend from tail ends of the corresponding three adjacent supporting sections 124 towards the interior of the fixing portion 120 to form a convergence section 125, by which the tail ends of the three adjacent supporting sections 124 are connected, such that the distal tail ends of the three supporting bodies 122 are connected to form a closed tail end. The three adjacent supporting sections 124 and the convergence section 125 connected thereto form a set of suspended sections 126, all of which are spaced apart and suspended along the circumferential direction of the fixing portion 120. For example, for the same set of suspended sections 126, a tail end of the first supporting section 124 may be connected to a tail end of the intermediate supporting section 124 and bent towards the interior of the fixing portion 120 so as to form a first connecting rod 1251 in V-shaped or U-shaped; a tail end of the second supporting section 124 may be connected to a tail end of the intermediate supporting section 124 and bent towards the interior of the fixing portion 120 so as to form a second connecting rod 1252 in V-shaped or U-shaped; and the first connecting rod 1251 and the second connecting rod 1252 are further connected by a third connecting rod 1253 in V-shaped or U-shaped. In other embodiments, it is also possible that four adjacent or more supporting bodies 122 bend and extend from tail ends of the corresponding four adjacent or more supporting sections 124 towards the interior of the fixing portion 120 to form a convergence section 125, which will not be repeated here. The fixing portion 120 in these embodiments has the same effect as the fixing portion 120 described above, which will not be repeated here.

### Embodiment 2

The occlusion device 100 in Embodiment 2 is generally similar to the occlusion device 100 in Embodiment 1, and the same features will not be repeated here. Embodiment 2 differs, for example, from Embodiment 1 in that the plurality of supporting sections 124 on the fixing portion 120 incline circumferentially around the central axis Y of the fixing portion 120, that is, the supporting sections 124 are neither parallel to nor coplanar with the central axis Y of the fixing portion 120. Therefore, it is possible to increase the contact area between the supporting sections 124 and pectinate muscles on the inner wall of the left atrial appendage and reduce the possibility of a part of the supporting sections 124 abutting against thin ravines between pectinate muscles, thereby reducing the probability of rupturing the left atrial appendage.

As shown in FIG. 7, the two adjacent supporting sections 124 are connected by the convergence section 125 to form the suspended section 126, and all the supporting sections 124 incline circumferentially to the same extent around the central axis Y of the fixing portion 120. Therefore, all the suspended sections 126 incline along the circumferential direction of the fixing portion 120 at intervals from each other in the same degree. In other embodiments, different supporting sections 124 or suspended sections 126 may incline along the circumferential direction of the fixing portion 120 in different degrees.

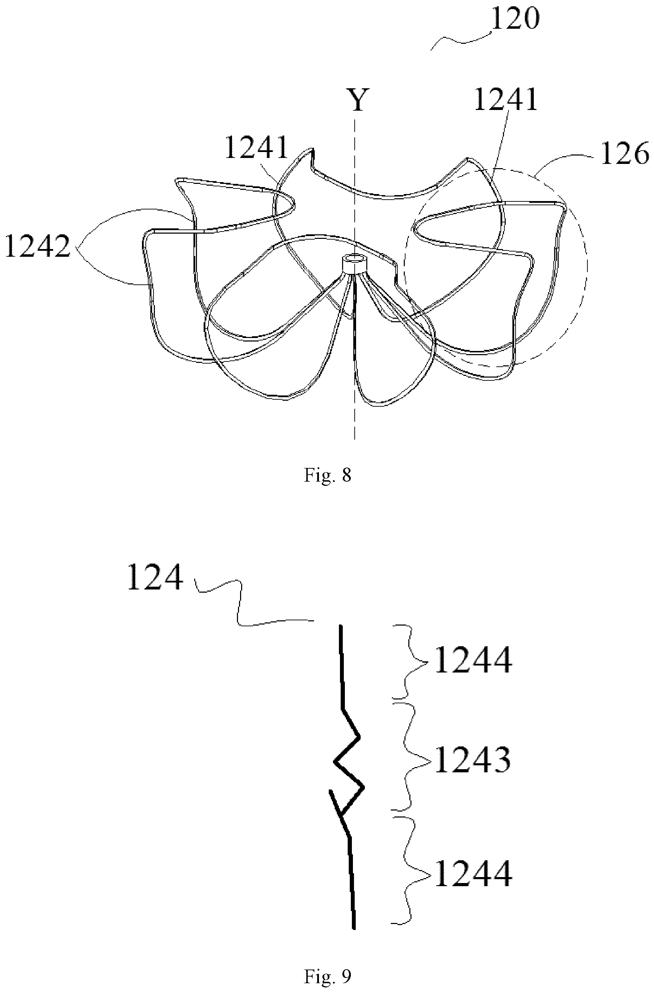

In other embodiments, at least two supporting sections 124, within at least one set of suspended sections 126 on the fixing portion 120, incline differently along the circumferential direction of the fixing portion 120. As shown in FIG. 8, the fixing portion 120 includes four suspended sections 126, where two supporting sections 1241 of at least one set of the suspended sections 126 incline differently along the circumferential direction of the fixing portion 120, for example, the two supporting sections 1241 are arranged in mirror symmetry; the two supporting sections 1242 of at least one set of the suspended sections 126 incline in the same direction along the circumferential direction of the fixing portion 120. The two supporting sections 124 within the same set of suspended sections 126, due to the different direction of inclination along the circumferential direction of the fixing portion 120, can avoid with a greater probability that a part of the supporting sections 124 abuts against the ravines between pectinate muscles, as compared to the same direction of inclination, greatly reducing the risk of rupturing the left atrial appendage.

In other embodiments, at least two adjacent sets of suspended sections 126 on the fixing portion 120 incline differently along the circumferential direction of the fixing portion 120. Further, the two sets of sections 126 may incline in different degrees. The fixing portion 120 can form a particular shape by virtue of the inclination of the two adjacent sets of suspended sections 126 in different directions and degrees, which can not only increase the contact area with the internal tissue, but also avoid with a greater probability that a part of the supporting section 124 abuts against the ravines between pectinate muscles, reducing the risk of rupturing the left atrial appendage to a greater extent.

### Embodiment 3

The occlusion device 100 in Embodiment 3 is generally similar to the occlusion device 100 in the Embodiments 1 and 2, and the same features will not be repeated here. Embodiment 3 differs, for example, from Embodiments 1 and 2 in that the supporting section 124 includes a first supporting area 1243 and a second supporting area 1244 connected to each other, and both the first supporting area 1243 and the second supporting area 1244 are compressed and constrained to be approximately straight in response to the fixing portion 120 being contained within the sheath. As shown in FIG. 9, upon release and deploying of the fixing portion 120, the first supporting area 1243 deforms along the circumferential direction of the fixing portion 120, and the second supporting area 1244 does not deform along the circumferential direction of the fixing portion 120. The maximum width of the first supporting area 1243 along the circumferential direction of the fixing portion 120 after deformation is greater than the maximum width of the second supporting area 1244 along the circumferential direction of the fixing portion 120. The first supporting area 1243 is generally a part where the fixing portion 120 contacts most with the internal tissue.

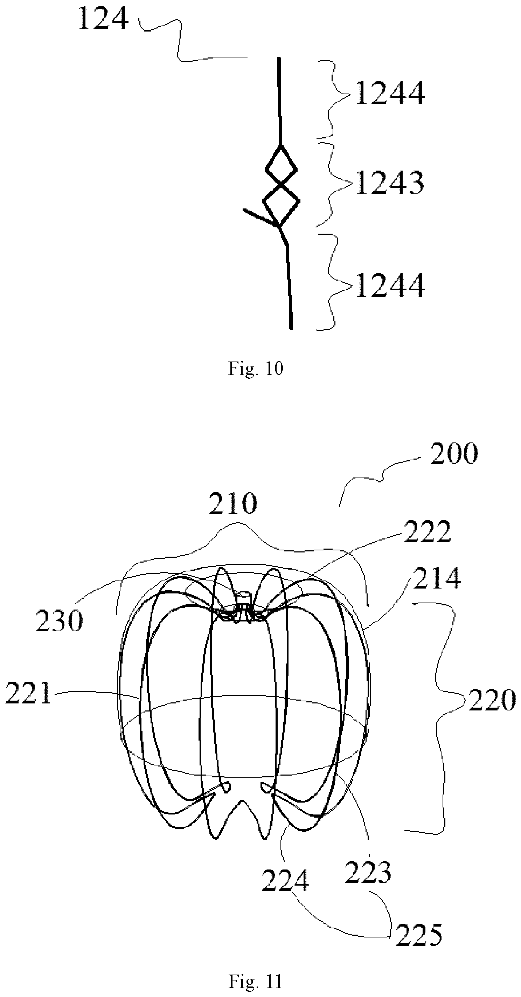

The deformation of the first supporting area 1243 may be a folded linear shape and a wave shape as shown in FIG. 9, a diamond shape or other polygonal shapes as shown in FIG. 10, or any combination of these shapes, the forms being not limited as long as the first supporting area 1243 has a larger maximum width along the circumferential direction of the fixing portion 120 than the second supporting area 1244 without deformation upon release of the fixing portion 120. The surface of the deformed first supporting area 1243 is an arc surface, which compared with the non-deformed second supporting area 1244, increases the contact area of the supporting section 124 with the internal tissue to improve the fixing stability of the fixing portion 120. Taking the left atrial appendage as an example, the deformed first supporting area 1243 not only increases the contact area between the supporting section 124 and the inner wall of the left atrial appendage, improving the stability of the fixing portion 120 in fixing the occlusion device 100 within the left atrial appendage; but also prevents the supporting section 124 from abutting against the weak ravines between pectinate muscles to a greater extent, due to the cooperation of the first supporting area 1243 and the second supporting area 1244 in which the first supporting area 1243 extends along the circumferential direction of the fixing portion 120 and the second supporting area 1244 extends along the axial direction Y of the fixing portion 120, thus greatly reducing the possibility of rupturing the ravines between pectinate muscles and avoiding the occurrence of pericardial effusion. In addition, it can also reduce the intensity of the convergence section 125 to withstand the impact of the inner wall of the left atrial appendage during the contraction and relaxation of the left atrial appendage.

Further, in other embodiments, the second supporting area 1244 also deforms along the circumferential direction of the fixing portion 120 upon release of the fixing portion 120. The deforming shape of the second supporting area 1244 may or may not be the same as that of the first supporting area 1243. Since the first supporting area 1243 is generally a region where the fixing portion 120 contacts most with the internal tissue, the maximum width of the second supporting area 1244 along the circumferential direction of the fixing portion 120 after deformation is generally smaller than the maximum width of the first supporting area 1243 along the circumferential direction of the fixing portion 120 after deformation. The specific width of both may be adjusted as desired, but is not limited thereto.

### Embodiment 4

An occlusion device 200 in Embodiment 4 is generally similar to the occlusion device 100 in the Embodiments 1, 2 and 3, and the same features will not be repeated here. Embodiment 4 differs, for example, from Embodiments 1, 2 and 3 in that each of a plurality of supporting bodies 221 of a fixing portion 220 has one end directly connected to a sealing portion 210 and the other end extending away from the sealing portion 210.

For example, as shown in FIG. 11, the fixing portion 220 and the sealing portion 210 of the occlusion device 200 are integrally formed. The occlusion device 200 includes a central end portion 230 at the proximal end, and a plurality of supporting bodies 221 surrounding and extending radially outward from the central end portion 230 towards the distal end. Each of the supporting bodies 221 includes a lead-out section 222 spreading radially outward from the central end portion 230, and a supporting section 223 connected with a distal end of the lead-out section 222 and extending towards the distal end. For two adjacent supporting bodies 221, their distal ends bend and extend from tail ends of the corresponding two adjacent supporting sections 223 towards the interior of the fixing portion 220, forming a convergence section 224. The two supporting sections 223 and the convergence section 224 connected thereto form a set of suspended sections 225, all of which are spaced apart and suspended along a circumferential direction of the fixing portion 220. The central end portion 230 can be detachably connected to a transporting rod. The occlusion device 200 is coated with at least one thin-film body 214 (the thin-film body 214 is transparent in FIG. 11 to facilitate viewing the structure of the supporting bodies 221), where the thin-film body 214 at least coats a proximal end of the occlusion device, for example, the thin-film body 214 coats a part of proximal ends of all the lead-out sections 222 and all the supporting sections 223, serving to occlude the opening of the internal tissue. All the lead-out sections 222 and the thin-film body 214 coating them form the sealing portion 210. All the supporting sections 223 and all the convergence section 224 form the fixing portion 220.

Compared with the related art, the suspended sections 225 retain the advantages of being independent from each other and with a large active space circumferentially of single supporting bodies circumferentially spaced and suspended, which can adaptively deform according to different internal tissue structures, and thus has flexible environmental adaptability. In addition, the suspended sections 225 enable tail ends of two adjacent supporting sections 223 to be connected to form a closed loop structure bending towards the interior of the fixing portion 220, which not only has a better anti-damage effect, but also can prevent a single supporting section 223 from being locally deformed to a large extent, and buffer the supporting section 223 with a single rod and ends not closed from greater pressure on the inner wall of the left atrial appendage, so as to enhance the fatigue resistance of the occlusion device, and at the same time, reduce the stimulation, wear, or even rupture to the inner wall of the left atrial appendage.

Although a distal end portion of each of the supporting bodies 221 in Embodiment 4 extends towards the distal direction, and the distal end portion of each of the supporting bodies 121 in Embodiments 1, 2 and 3 extends towards the sealing portion 110 (that is, towards the proximal direction), the supporting sections 223 in Embodiment 4 may also adopt the relevant solutions of inclining circumferentially around the central axis Y of the fixing portion 120 in Embodiment 2 and the relevant solutions of the first supporting area 1243 in Embodiment 3, but not limited thereto. Unless there is no conflict in the combination of features in different embodiments of any of the above-mentioned embodiments, the description thereof will not be repeated here.

Each of the features of the above-mentioned embodiments can be arbitrarily combined. For the sake of concise description, all possible combinations of each of the features in the above-mentioned embodiments have not been described. However, unless there is no contradiction between the combinations of these features, they should be considered within the scope of the embodiments.

The embodiments described above, with more specific and detailed descriptions, represent only several embodiments, which cannot be interpreted as a limitation on the scope of the embodiments. It should be noted that for those ordinarily skilled in the art, several changes and improvements can be further made without deviating from the principle of the embodiments, which shall fall within the scope of the present embodiments.

## Claims

1. An occlusion device (100), comprising:
a fixing portion (120), the fixing portion comprising a plurality of supporting bodies (122), the plurality of supporting bodies (122) comprising a plurality of supporting sections (124) spaced apart along a circumferential direction of the fixing portion (120);
for all of the plurality of supporting sections (124), tail ends of at least two adjacent supporting sections (124) bend and extend towards the interior of the fixing portion (120) to form a convergence section (125), by which the tail ends of the at least two adjacent supporting sections (124) are connected; and wherein the at least two adjacent supporting sections (124) and the corresponding convergence section (125) form a set of suspended section (126), all sets of suspended sections (126) are spaced apart, suspended along the circumferential direction of the fixing portion (120) and being independent from each other.

2. The occlusion device (100) according to claim 1, wherein the plurality of supporting sections (124) inclines circumferentially around a central axis of the fixing portion (120).

3. The occlusion device (100) according to claim 2, wherein at least two of the supporting sections (124) within at least one set of the suspended sections (126) incline in the same or different directions along the circumferential direction of the fixing portion (120).

4. The occlusion device according to claim 2, wherein at least two adjacent sets of the suspended sections incline in different directions along the circumferential direction of the fixing portion.

5. The occlusion device (100) according to claim 1, wherein each of the supporting sections (124) comprises a first supporting area (1243) and a second supporting area (1244) connected to each other; wherein the first supporting area (1243) deforms along the circumferential direction of the fixing portion (120) upon release and spreading of the fixing portion (120); and wherein a maximum width of the first supporting area (1243) along the circumferential direction of the fixing portion (120) after deformation is greater than a maximum width of the second supporting area (1244) along the circumferential direction of the fixing portion (120).

6. The occlusion device (100) according to claim 5, wherein the second supporting area (1244) deforms along the circumferential direction of the fixing portion (120) upon a release of the fixing portion (120).

7. The occlusion device (100) according to claim 1, wherein an innermost end portion (1253) of the convergence section (125) is connected with an internally hollow woven mesh (1254).

8. The occlusion device (100) according to claim 1, wherein the occlusion device (100) further comprises a sealing portion (110) connected to the fixing portion (120), wherein one end of each of the plurality of supporting bodies (122) is connected to the sealing portion (110) and the other end extends away from the sealing portion (110).

9. The occlusion device (100) according to claim 1, wherein the fixing portion (120) further comprises a central end portion (121), and the occlusion device (100) further comprises a sealing portion (110) connected to the central end portion (121) and disposed at one side of the fixing portion (120); wherein one end of each of the plurality of supporting bodies (122) is connected to the central end portion (121), and the other end extends radially outward from the central end portion (121) and flipping towards the sealing portion (110) to form a flipping section(123); and wherein each of the plurality of supporting bodies (122) continues to extend from a tail end of the flipping section (123) towards the sealing portion (110) to form the supporting section (124).

10. The occlusion device (100) according to claim 9, wherein the sealing portion (110) comprises a distal disc surface (112) facing the fixing portion (120), at least a part of the distal disc surface (112) being coated with at least one thin-film body (114).

## Patentansprüche

1. Verschlussvorrichtung (100), umfassend:
einen Befestigungsteil (120), wobei der Befestigungsteil eine Vielzahl von Stützkörpern (122) umfasst, wobei die Vielzahl von Stützkörpern (122) eine Vielzahl von Stützabschnitten (124) umfasst, die entlang einer Umfangsrichtung des Befestigungsteils (120) voneinander beabstandet sind;
wobei bei allen der Vielzahl von Stützabschnitten (124) die hinteren Enden von wenigstens zwei benachbarten Stützabschnitten (124) sich biegen und sich zum Inneren des Befestigungsteils (120) hin erstrecken, um einen Konvergenzabschnitt (125) zu bilden, durch den die hinteren Enden der wenigstens zwei benachbarten Stützabschnitte (124) miteinander verbunden sind; und wobei die wenigstens zwei benachbarten Stützabschnitte (124) und der entsprechende Konvergenzabschnitt (125) einen Satz von Hängeabschnitten (126) bilden,
wobei alle Sätze von Hängeabschnitten (126) voneinander beabstandet sind, entlang der Umfangsrichtung des Befestigungsteils (120) aufgehängt sind und voneinander unabhängig sind.

2. Verschlussvorrichtung (100) nach Anspruch 1, wobei die Vielzahl von Stützabschnitten (124) sich um eine Mittelachse des Befestigungsteils (120) herum in Umfangsrichtung neigt.

3. Verschlussvorrichtung (100) nach Anspruch 2, wobei wenigstens zwei der Stützabschnitte (124) innerhalb wenigstens eines Satzes der Hängeabschnitte (126) sich in gleiche oder unterschiedliche Richtungen entlang der Umfangsrichtung des Befestigungsteils (120) neigen.

4. Verschlussvorrichtung nach Anspruch 2, wobei wenigstens zwei benachbarte Sätze von den Hängeabschnitten sich in unterschiedlichen Richtungen entlang der Umfangsrichtung des Befestigungsteils neigen.

5. Verschlussvorrichtung (100) nach Anspruch 1, wobei jeder der Stützabschnitte (124) einen ersten Stützbereich (1243) und einen zweiten Stützbereich (1244) umfasst, die miteinander verbunden sind; wobei sich der erste Stützbereich (1243) bei Freisetzen und Ausbreiten des Befestigungsteils (120) entlang der Umfangsrichtung des Befestigungsteils (120) verformt; und wobei eine maximale Breite des ersten Stützbereichs (1243) entlang der Umfangsrichtung des Befestigungsteils (120) nach der Verformung größer ist als eine maximale Breite des zweiten Stützbereichs (1244) entlang der Umfangsrichtung des Befestigungsteils (120).

6. Verschlussvorrichtung (100) nach Anspruch 5, wobei sich der zweite Stützbereich (1244) bei einem Freisetzen des Befestigungsteils (120) entlang der Umfangsrichtung des Befestigungsteils (120) verformt.

7. Verschlussvorrichtung (100) nach Anspruch 1, wobei ein innerster Endteil (1253) des Konvergenzabschnitts (125) mit einem innen hohlen Geflecht (1254) verbunden ist.

8. Verschlussvorrichtung (100) nach Anspruch 1, wobei die Verschlussvorrichtung (100) ferner einen mit dem Befestigungsteil (120) verbundenen Dichtungsteil (110) umfasst, wobei ein Ende jedes der Vielzahl von Stützkörpern (122) mit dem Dichtungsteil (110) verbunden ist und sich das andere Ende vom Dichtungsteil (110) weg erstreckt.

9. Verschlussvorrichtung (100) nach Anspruch 1, wobei der Befestigungsteil (120) ferner einen zentralen Endteil (121) umfasst und die Verschlussvorrichtung (100) ferner einen Dichtungsteil (110) umfasst, der mit dem zentralen Endteil (121) verbunden ist und an einer Seite des Befestigungsteils (120) angeordnet ist; wobei ein Ende jedes der Vielzahl von Stützkörpern (122) mit dem zentralen Endteil (121) verbunden ist und das andere Ende sich radial nach außen vom zentralen Endteil (121) erstreckt und in Richtung des Dichtungsteils (110) umklappt, um einen Umschlagabschnitt (123) zu bilden; und wobei sich jeder der Vielzahl von Stützkörpern (122) von einem hinteren Ende des Umschlagabschnitts (123) weiter in Richtung des Dichtungsteils (110) erstreckt, um den Stützabschnitt (124) zu bilden.

10. Verschlussvorrichtung (100) nach Anspruch 9, wobei der Dichtungsteil (110) eine dem Befestigungsteil (120) zugewandte distale Scheibenfläche (112) umfasst, wobei wenigstens ein Teil der distalen Scheibenfläche (112) mit wenigstens einem Dünnschichtkörper (114) beschichtet ist.

## Revendications

1. Dispositif d'occlusion (100) comprenant :
une partie de fixation (120), la partie de fixation comprenant une pluralité de corps de support (122), la pluralité de corps de support (122) comprenant une pluralité de sections de support (124) espacées le long de la direction circonférentielle de la partie de fixation (120) ;
pour l'ensemble de la pluralité de sections de support (124), les extrémités de queue d'au moins deux sections de support adjacentes (124) se plient et s'étendent vers l'intérieur de la partie de fixation (120) de manière à former une section de convergence (125), par laquelle les extrémités de queue desdites au moins deux sections de support adjacentes (124) sont reliées ; et lesdites au moins deux sections de support adjacentes (124) et ladite section de convergence correspondante (125) formant un ensemble de section suspendue (126), tous les ensembles de sections suspendues (126) étant espacés, suspendus le long de la direction circonférentielle de la partie de fixation (120) et étant indépendants les uns des autres.

2. Dispositif d'occlusion (100) selon la revendication 1, ladite pluralité de sections de support (124) s'inclinant de manière circonférentielle autour de l'axe central de la partie de fixation (120).

3. Dispositif d'occlusion (100) selon la revendication 2, au moins deux des sections de support (124) à l'intérieur d'au moins un ensemble des sections suspendues (126) s'inclinant dans la même direction ou dans des directions différentes le long de la direction circonférentielle de la partie de fixation (120).

4. Dispositif d'occlusion selon la revendication 2, au moins deux ensembles adjacents des sections suspendues s'inclinant dans des directions différentes le long de la direction circonférentielle de la partie de fixation.

5. Dispositif d'occlusion (100) selon la revendication 1, chacune des sections de support (124) comprenant une première zone de support (1243) et une seconde zone de support (1244) reliées l'une à l'autre ; ladite première zone de support (1243) se déformant le long de la direction circonférentielle de la partie de fixation (120) lors de la libération et de l'étalement de la partie de fixation (120) ; et la largeur maximale de la première zone de support (1243) le long de la direction circonférentielle de la partie de fixation (120) après déformation étant supérieure à la largeur maximale de la seconde zone de support (1244) le long de la direction circonférentielle de la partie de fixation (120).

6. Dispositif d'occlusion (100) selon la revendication 5, ladite seconde zone de support (1244) se déformant le long de la direction circonférentielle de la partie de fixation (120) lors de la libération de la partie de fixation (120).

7. Dispositif d'occlusion (100) selon la revendication 1, la partie d'extrémité la plus interne (1253) de la section de convergence (125) étant reliée à une maille tissée intérieurement creuse (1254).

8. Dispositif d'occlusion (100) selon la revendication 1, ledit dispositif d'occlusion (100) comprenant en outre une partie d'étanchéité (110) reliée à la partie de fixation (120), une extrémité de chacun de la pluralité de corps de support (122) étant reliée à la partie d'étanchéité (110) et l'autre extrémité s'étendant à l'opposé de la partie d'étanchéité (110).

9. Dispositif d'occlusion (100) selon la revendication 1, ladite partie de fixation (120) comprenant en outre une partie d'extrémité centrale (121), et ledit dispositif d'occlusion (100) comprenant en outre une partie d'étanchéité (110) reliée à la partie d'extrémité centrale (121) et disposée au niveau d'un côté de la partie de fixation (120) ; une extrémité de chacun de la pluralité de corps de support (122) étant reliée à la partie d'extrémité centrale (121), et l'autre extrémité s'étendant radialement vers l'extérieur à partir de la partie d'extrémité centrale (121) et basculant vers la partie d'étanchéité (110) de manière à former une section de basculement (123) ; et chacun de la pluralité de corps de support (122) continuant de s'étendre à partir de l'extrémité arrière de la section de basculement (123) vers la partie d'étanchéité (110) de manière à former la section de support (124).

10. Dispositif d'occlusion (100) selon la revendication 9, ladite partie d'étanchéité (110) comprenant une surface de disque distale (112) faisant face à la partie de fixation (120), au moins une partie de la surface de disque distale (112) étant revêtue d'au moins un corps de film mince (114).
